(19) Europäisches Patentamt
European Patent Office
Office européen des brevets

(11) Publication number: 0 214 872 A2

# (12) EUROPEAN PATENT APPLICATION

(21) Application number: 86307044.7

(22) Date of filing: 12.09.86

(51) Int. Cl.⁴: C 07 K 7/06
C 07 K 7/26, A 61 K 37/02

(30) Priority: 12.09.85 US 775488
17.06.86 US 875266

(43) Date of publication of application: 18.03.87 Bulletin 87/12

(84) Designated Contracting States: AT BE CH DE FR GB IT LI LU NL SE

(71) Applicant: The Administrators of The Tulane Educational Fund
1430 Tulane Avenue
New Orleans Louisiana 70112(US)

(72) Inventor: Coy, David H.
4319 Perrier Street
New Orleans Louisiana 70115(US)

(72) Inventor: Murphy, William A.
Route 8, Box 979
Covington Louisiana 70443(US)

(72) Inventor: Heiman, Mark L.
2513 Jasmine Street
New Orleans Louisiana 70122(US)

(74) Representative: Sheard, Andrew Gregory et al,
Kilburn & Strode 30, John Street
London WC1N 2DD(GB)

## (54) Somatostatin analoge.

(57) Octapeptides of the formula:

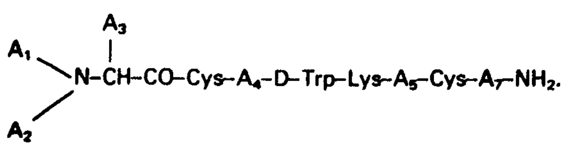

wherein each $A_1$ and $A_2$, independently, is H, $C_{1-12}$ alkyl, $C_{7-10}$ phenylalkyl, $R_1CO$ (where $R_1$ is $C_{1-20}$ alkyl, $C_{3-20}$ alkenyl, $C_{3-20}$ alkynyl, phenyl, naphthyl, or $C_{7-10}$ phenylalkyl), or $R_2OCO$ (where $R_2$ is $C_{1-10}$ alkyl or $C_{7-10}$ phenylalkyl), provided that when one of $A_1$ or $A_2$ is $R_1CO$ or $R_2OCO$, the other must be H; $A_3$ is $CH_2$-$A_6$ (where $A_6$ is pentafluorophenyl, naphthyl, pyridyl, or phenyl); $A_4$ is o- m- or p-substituted X-Phe (where X is halogen, H, $NO_2$, OH, $NH_2$, or $C_{1-3}$ alkyl), pentafluoro-Phe, or beta-Nal; $A_5$ is Thr, Ser, Phe, Val, alpha-aminoisobutyric acid, or Ile, provided that when $A_3$ is phenyl, $A_1$ is H, and $A_2$ is H, $A_6$ cannot be Val; and $A_7$ is Thr, Trp, or beta-Nal; and their salts are useful in the reduction of growth hormone, insulin, glucagon, or pancreatic exocrine secretion.

EP 0 214 872 A2

Croydon Printing Company Ltd.

# SOMATOSTATIN ANALOGUE

This invention relates to therapeutic peptides.

A number of somatostatin analogues exhibiting GH-release-inhibiting activity have been described in the literature, including analogues containing fewer than the naturally occurring fourteen amino acids. For example, Coy et al. U.S. Patent No. 4,485,101, hereby incorporated by reference, describes dodecapeptides having an N-terminal acetyl group, a C-terminal $NH_2$, D-Trp at position 6, and p-Cl-Phe at position 4. (Herein, when no designation of configuration is given, the L-isomer is intended.)

In general, the invention features an octapeptide of the formula:

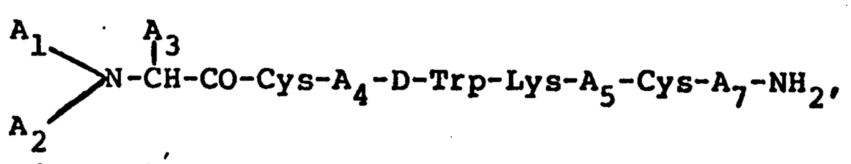

wherein each $A_1$ and $A_2$, independently, is H, $C_{1-12}$ alkyl, $C_{7-10}$ phenylalkyl, $R_1CO$ (where $R_1$ is $C_{1-20}$ alkyl, $C_{3-20}$ alkenyl, $C_{3-20}$ alkynyl, phenyl, naphthyl, or $C_{7-10}$ phenylalkyl), or $R_2OCO$ (where $R_2$ is $C_{1-10}$ alkyl or $C_{7-10}$ phenylalkyl), provided that when one of $A_1$ or $A_2$ is $R_1CO$ or $R_2OCO$, the other must be H; $A_3$ is $CH_2$-$A_6$ (where $A_6$ is pentafluorophenyl, naphthyl, pyridyl, or phenyl); $A_4$ is o- m- or, more preferably, p-substituted X-Phe (where X is a halogen, H, $NH_2$, $NO_2$, OH, or $C_{1-3}$ alkyl), pentafluoro-Phe, or β-Nal; $A_5$ is Thr, Ser, Phe, Val, α-aminoisobutyric acid, or Ile, provided that when $A_3$ is phenyl, $A_1$ is H, and $A_2$ is H, $A_5$ cannot be

Val; and $A_7$ is Thr, Trp, or β-Nal; or a pharmaceutically acceptable salt thereof.

In the formula given above, the configuration of the molecule at the carbon atom to which $A_3$ is bonded is not given, to indicate that the amino acid residue of which $A_3$ is a substituent can have the D- or L- configuration.

Preferred compounds of the invention include D-β-Nal-Cys-Tyr-D-Trp-Lys-Val-Cys-Thr-$NH_2$; D-Phe-Cys-Tyr-D-Trp-Lys-α-Aminoisobutyric acid-Cys-Thr-$NH_2$; pentafluoro-D-Phe-Cys-Tyr-D-Trp-Lys-Val-Cys-Thr-$NH_2$; N-Ac-D-β-Nal-Cys-Tyr-D-Trp-Lys-Val-Cys-Thr-$NH_2$; D-β-Nal-Cys-pentafluoro-Phe-D-Trp-Lys-Val-Cys-Thr-$NH_2$; D-β-Nal-Cys-Tyr-D-Trp-Lys-Val-Cys-β-Nal-Thr-$NH_2$; D-Phe-Cys-Tyr-D-Trp-Lys-Val-Cys-β-Nal-Thr-$NH_2$; and D-β-Nal-Cys-Tyr-D-Trp-Lys-α-aminoisobutyric acid-Cys-Thr-$NH_2$.

In other preferred embodiments, a therapeutically effective amount of the therapeutic compound and a pharmaceutically acceptable carrier substance, e.g. magnesium carbonate, lactose, or a phospholipid with which the therapeutic compound can form a micelle, together form a therapeutic composition, e.g. a pill, tablet, capsule, or liquid for oral administration to a human patient, a spreadable cream, gel, lotion, or ointment for application to the skin of a human patient in need of the compound, a liquid capable of being administered nasally as drops or spray, or a liquid capable of intravenous, parenteral, subcutaneous, or intraperitoneal administration. The pill, tablet or capsule can be coated with a substance capable of protecting the composition from the gastric acid in the patient's stomach for a period of time

sufficient to allow the composition to pass undisintegrated into the patient's small intestine. The therapeutic composition can also be in the form of a biodegradable sustained release formulation for intramuscular administration. For maximum efficacy, zero order release is desired. Zero order release can be obtained using an implantable or external pump, e.g., Infusoid[TM] pump, to administer the therapeutic composition.

The compounds of the invention are active in inhibiting the secretion of GH, insulin, and glucagon. Further, the aromatic lipophilic N-terminal end can provide long-lasting *in vivo* activity.

Other features and advantages of the invention will be apparent from the following description of the preferred embodiments thereof, and from the claims.

Structure

The compounds of the invention have the general formula recited above.
They are all octapeptide analogs of somatostatin which have D-Trp at position 4; and optional modifications at positions 3 ($A_4$) 6 ($A_5$) and 8($A_7$). It has been found that D- β -naphthylalanine at position 1; Tyr at position 3; and Val at position 6 are modifications which particularly enhance activity.

The compounds can be provided in the form of pharmaceutically acceptable salts. Examples of preferred salts are those with therapeutically acceptable organic acids, e.g., acetic, lactic, maleic, citric, malic, ascorbic, succinic, benzoic, salicylic, methanesulphonic, toluenesulphonic, or pamoic acid, as well as polymeric acids such as tannic acid or carboxymethyl cellulose, and salts with inorganic acids

such as the hydrohalic acids, e.g., hydrochloric acid, sulfuric acid, or phosphoric acid.

Synthesis

An exemplary synthesis of one octapeptide follows. Other octapeptides of the invention can be prepared by making appropriate modifications, within the ability of someone of ordinary skill in this field, of the following synthetic method.

The first step in the preparation of D- β -naphthylalanine-Cys-Tyr-D-Trp-Lys-Val-Cys-Thr-$NH_2$ was the preparation of the intermediate tert-butyloxycarbonyl-D- β -naphthylalanic-S-methylbenzyl-Cys-Tyr-D-Trp-$N^E$-benzyloxycarbonyl-Lys-Val-S-methylbenzyl-Cys-O-benzyl-Thr-benzyhydrylaminic resin, as follows.

Benzhydrylamine-polystyrene resin (Vega Biochemicals, Inc.) in the chloride ion form was placed in the reaction vessel of a Beckman 990B peptide synthesizer programmed to perform the following reaction cycle: (a) methylene chloride; (b) 33% trifluoroacetic acid in methylene chloride (2 times for 1 and 25 min each); (c) methylene chloride; (d) ethanol; (e) methylene chloride; (f) 10% triethylamine in chloroform.

The neutralized resin was stirred with Boc-O-benzyl-threonine and diisopropylcarbodiimide (1.5 mmole each) in methylene chloride for 1 h and the resulting amino acid resin was then cycled through steps (a) to (g) in the above wash program. The following amino acids (1.5 mmole) were then coupled successively by the same procedure: Boc-S-methylbenzyl-Cys, Boc-Val, Boc-NE-benzyloxycarbonyl-lysine, Boc-D-Trp, Boc-Tyr, Boc-S-methylbenzyl-Cys, Boc-D- β -naphthylalanine.

The resin was washed and dried and then mixed with anisole (4 ml) and anhydrous hydrogen fluoride (36

ml) at 0°C and stirred for 45 min. Excess hydrogen fluoride was evaporated rapidly under a stream of dry nitrogen and free peptide precipitated and washed with ether. The crude peptide was then dissolved in 800 ml of 90% acetic acid to which was added $I_2$ in methanol until a permanent brown colour was present. The solution was then stirred for 1 h before removing the solvent in vacuo. The resulting oil was dissolved in a minimum volume of 50% acetic acid and eluted on a column (2.5 X 100 mm) of Sephadex G-25. Fractions containing a major component by uv absorption and thin layer chromatography were then pooled, evaporated to a small volume, and applied to a column (2.5 X 50 cm) of Whatman LRP-1 octadecylsilane (15-20 μM).

The column was eluted with a linear gradient of 10-50% acetonitrile in 0.1% trifluoroacetic acid in water. Fractions were examined by thin layer chromatography and analytical high performance liquid chromatography and pooled to give maximum purity and if desired, a different salt prepared, e.g., acetate or phosphate. Repeated lyophilization of the solution from water gave 170 mg of the product as a white, fluffy powder.

The product was found to be homogeneous by Hplc and Tlc. Amino acid analysis of an acid hydrolysate confirmed the composition of the octapeptide.

The octapeptides of the invention having the formulae pentafluoro-D-Phe-Cys-Tyr-D-Trp-Lys-Val-Cys-Thr-$NH_2$, D-Phe-Cys-Tyr-D-Trp-Lys-α-aminoisobutyric acid-Cys-Thr-$NH_2$, N-Ac-D-β-Nal-Cys-Tyr-D-Trp-Lys-Val-Cys-Thr-$NH_2$, D-β-Nal-Cys-pentafluoro-Phe-D-Trp-Lys-Val-Cys-Thr-$NH_2$, D- β -Nal-Cys-Tyr-D-Trp-Lys-Val-Cys- β -Nal-Thr-$NH_2$, D-Phe-Cys-Tyr-D-Trp-Lys-Val-Cys- β -Nal-Thr- $NH_2$; and D- β -Nal-Cys-Tyr-D-Trp-Lys- α -aminoisobutyric acid-Cys-Thr-$NH_2$ were made according to methods analogous to those described above.

Use

When administered to mammals, particularly humans, (e.g. orally, topically, intravenously, parenterally in a sustained release, biodegradable form, nasally, or by suppository), the compounds can be effective to inhibit GH release as well as to inhibit insulin, glucagon, and pancreatic exocrine secretion, and to therapeutically affect the central nervous system.

The compounds can be administered to a mammal, e.g. a human, in the dosages used for somatostatin or, because of their greater potency, in smaller dosages. The compounds of the invention can be used for the treatment of cancer, particularly growth hormone-dependent cancer (e.g., bone, cartilage, pancreas (endocrine and exocrine), prostate, or breast), acromegaly and related hypersecretroy endocrine states, or of bleeding ulcers in emergency patients and in those suffering from pancreatitis or diarrhoea. The compounds can also be used in the management of diabetes and to protect the liver of patients suffering from cirrhosis or hepatitis. The compounds can also be used to treat Alzheimer's disease, as analgesics to treat pain by acting specifically on certain opiate receptors, and as gastric cytoprotective compounds for ulcer therapy. The compounds can also be used to treat certain types of mushroom poisoning.

The compounds can also be used to treat diabetes-related retinopathy. The anti-cancer activity

of the compounds may be related to their ability to antagonize cancer-related growth factors such as epidermal growth factor.

The compounds can be administered to a mammal, e.g., a human, in a dosage of 0.01 to 1000 mcg/kg/day, preferably 0.1 to 100 mcg/kg/day.

Other embodiments are within the following claims.

CLAIMS FOR THE CONTRACTING STATES: BE, FR, DE, IT, LU, NL, SE, CH, LI and GB.

1. An octapeptide of the formula:

$$\begin{matrix} A_1 & A_3 \\ & | \\ & N\text{-}CH\text{-}CO\text{-}Cys\text{-}A_4\text{-}D\text{-}Trp\text{-}Lys\text{-}A_5\text{-}Cys\text{-}A_7\text{-}NH_2, \\ A_2 & \end{matrix}$$

wherein each $A_1$ and $A_2$, independently, is H, $C_{1-12}$ alkyl, $C_{7-10}$phenylalkyl, $R_1CO$ (where $R_1$ is $C_{1-20}$ alkyl, $C_{3-20}$ alkenyl, $C_{3-20}$ alkynyl, phenyl, naphthyl, or $C_{7-10}$ phenylalkyl), or $R_2OCO$ (where $R_2$ is $C_{1-10}$ alkyl or $C_{7-10}$ phenylalkyl), provided that when one of $A_1$ or $A_2$ is $R_1CO$ or $R_2OCO$, the other must be H; $A_3$ is $CH_2\text{-}A_6$ (where $A_6$ is pentafluorophenyl, naphthyl, pyridyl, or phenyl); $A_4$ is o- m- or p-substituted X-Phe (where X is a halogen, H, $NO_2$, OH, $NH_2$, or $C_{1-3}$ alkyl), pentafluoro-Phe, or beta-Nal; $A_5$ is Thr, Ser, Phe, Val, alpha-aminoisobutyric acid, or Ile, provided that when $A_3$ is phenyl, $A_1$ is H, and $A_2$ is H, $A_5$ cannot be Val; and $A_7$ is Thr, Trp, or beta-Nal; or a pharmaceutically acceptable salt thereof.

2. An octapeptide as claimed in claim 1 wherein

$$\begin{matrix} A_1 A_3 \\ N\text{-}CH\text{-}CO \\ A_2 \end{matrix}$$ is D-beta-naphthylalanine.

3. An octapeptide as claimed in claim 1 wherein

$$\begin{matrix} A_1 A_3 \\ N\text{-}CH\text{-}CO \\ A_2 \end{matrix}$$ is D-Phe and $A_5$ is alpha-aminoisobutyric acid.

4. An octapeptide as claimed in claim 1, 2 or 3, wherein $A_6$ is naphthyl or pentafluorophenyl.

5. An octapeptide as claimed in any one of claims 1 to 4, wherein $R_1$ is $CH_3$ or $C_2H_5$.

6. D-beta-Nal-Cys-Tyr-D-Trp-Lys-Val-Cys-Thr-$NH_2$, pentafluoro-D-Phe-Cys-Tyr-D-Trp-Lys-Val-Cys-Thr-$NH_2$, D-Phe-Cys-Tyr-D-Trp-Lys-alpha-aminoisobutyric acid-Cys-Thr-$NH_2$, N-Ac-D-beta-Nal-Cys-Tyr-D-Trp-Lys-Val-Cys-Thr-$NH_2$, D-beta-Nal-Cys-pentafluoro-Phe-D-Trp-Lys-Val-Cys-Thr-$NH_2$, D-beta-Nal-Cys-Tyr-D-Trp-Lys-Val-Cys-beta-Nal-Thr-$NH_2$, D-Phe-Cys-Tyr-D-Trp-Lys-Val-Cys-beta-Nal-Thr-$NH_2$, D-beta-Nal-Cys-Tyr-D-Trp-Lys-alpha-aminoisobutyric acid-Cys-Thr-$NH_2$ or a pharmaceutically acceptable salt of any of these compounds.

7. A therapeutic composition capable of inhibiting the release of growth hormone, insulin, glucagon, or pancreatic exocrine secretion comprising a therapeutically effective amount of the compound as claimed in any one of claims 1 to 6 together with a pharmaceutically acceptable carrier substance.

8. The therapeutic composition of claim 7 wherein said composition is in the form of:

a pill, tablet, or capsule for oral administration to a human patient in need of said compound;

a liquid for oral administration to a human patient in

need of said compound;

a cream, gel, spray, or ointment for application to the skin of a human patient in need of said compound;

a liquid capable of being administered nasally as drops or spray to a human patient in need of said compound;

a liquid for intravenous, subcutaneous, parenteral, or intraperitioneal administration to a human patient in need of said compound; or

a biodegradable sustained release composition for intramuscular administration to a human patient in need of said compound.

9. A therapeutic composition as claimed in claim 8, said composition being a pill, tablet or capsule for human oral administration coated with a substance capable of protecting said composition from the gastric acid in the stomach of said human patient for a period of time sufficient to allow said composition to pass undisintegrated into the small intestine of said human patient.

10. An octapeptide as claimed in any one of claims 1 to 6 for use in medicine.

11. The use of an octapeptide as claimed in any one of claims 1 to 6 in the preparation of a medicament for the reduction of growth hormone, insulin, glucagon, or pancreatic exocrine secretion.

CLAIMS FOR THE CONTRACTING STATE: AT

1. A process for the preparation of an octapeptide of the formula:

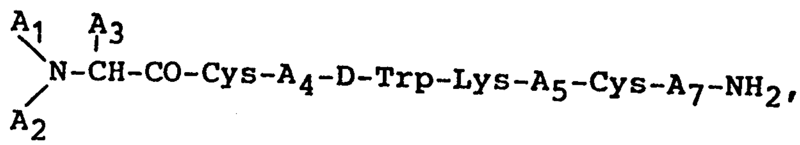

wherein each $A_1$ and $A_2$, independently, is H, $C_{1-12}$ alkyl, $C_{7-10}$phenylalkyl, $R_1CO$ (where $R_1$ is $C_{1-20}$ alkyl, $C_{3-20}$ alkenyl, $C_{3-20}$ alkynyl, phenyl, naphthyl, or $C_{7-10}$ phenylalkyl), or $R_2OCO$ (where $R_2$ is $C_{1-10}$ alkyl or $C_{7-10}$ phenylalkyl), provided that when one of $A_1$ or $A_2$ is $R_1CO$ or $R_2OCO$, the other must be H; $A_3$ is $CH_2$-$A_6$ (where $A_6$ is pentafluorophenyl, naphthyl, pyridyl, or phenyl); $A_4$ is o- m- or p-substituted X-Phe (where X is a halogen, H, $NO_2$, OH, $NH_2$, or $C_{1-3}$ alkyl), pentafluoro-Phe, or beta-Nal; $A_5$ is Thr, Ser, Phe, Val, alpha-aminoisobutyric acid, or Ile, provided that when $A_3$ is phenyl, $A_1$ is H, and $A_2$ is H, $A_5$ cannot be Val; and $A_7$ is Thr, Trp, or beta-Nal; or a pharmaceutically acceptable salt thereof; the process comprising adding successive optionally substituted amino acid residues, as defined above, to the N-terminal optionally substituted amino acid, or a residue thereof, and if necessary subsequently working up the reaction product to form the free octapeptide or a pharmaceutically acceptable salt thereof.

2. A process as claimed in claim 1 wherein 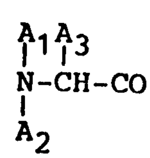 is D-beta-naphthylalanine.

3. A process as claimed in claim 1 wherein 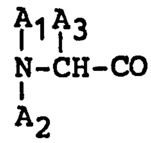 is D-Phe and $A_5$ is alpha-aminoisobutyric acid.

4. A process as claimed in claim 1, 2 or 3, wherein $A_6$ is naphthyl or pentafluorophenyl.

5. A process as claimed in any one of claims 1 to 4, wherein $R_1$ is $CH_3$ or $C_2H_5$.

6. A process for the preparation of
D-beta-Nal-Cys-Tyr-D-Trp-Lys-Val-Cys-Thr-$NH_2$,
pentafluoro-D-Phe-Cys-Tyr-D-Trp-Lys-Val-Cys-Thr-$NH_2$,
D-Phe-Cys-Tyr-D-Trp-Lys-alpha-aminoisobutyric acid-Cys-Thr-$NH_2$,
N-Ac-D-beta-Nal-Cys-Tyr-D-Trp-Lys-Val-Cys-Thr-$NH_2$,
D-beta-Nal-Cys-pentafluoro-Phe-D-Trp-Lys-Val-Cys-Thr-$NH_2$,
D-beta-Nal-Cys-Tyr-D-Trp-Lys-Val-Cys-beta-Nal-Thr-$NH_2$,
D-Phe-Cys-Tyr-D-Trp-Lys-Val-Cys-beta-Nal-Thr-$NH_2$,
D-beta-Nal-Cys-Tyr-D-Trp-Lys-alpha-aminoisobutyric acid-Cys-Thr-$NH_2$ or a pharmaceutically acceptable salt thereof;

the process comprising adding successive optionally substituted amino acid residues, as defined above, to the N-terminal optionally substituted amino acid, or a residue thereof, and if necessary subsequently working up the reaction product to form the free octapeptide or a pharmaceutically acceptable salt thereof.